# DEMANDE DE BREVET EUROPEEN

(11) **EP 1 323 397 A2**
(43) Date de publication de la demande: **02.07.2003**
(21) Numéro de dépôt: 02356253.1
(22) Date de dépôt: 06.12.2002
(51) Int. Cl.: A61F 13/08

(54) **Bas de contention destiné à être utilisé pour protéger ou soulager l'articulation du genou et/ou l'articulation de la cheville**

(30) Priorité: 07.12.2001 FR 0115876
(71) Demandeur: Laurencon, Michel, 69300 Caluire et Cuire (FR)
(72) Inventeur: Laurencon, Michel, 69300 Caluire et Cuire (FR)
(74) Mandataire: Guerre, Dominique

(57) **Abrégé**

Ce bas (1) comprend :
- une enveloppe (2), et
- une bande de renforcement (3) solidaire de l'enveloppe (2), comprenant une partie médiane (3a) qui passe, lorsque le bas (1) est porté, sous le pied (12) de l'utilisateur au niveau de la voûte plantaire, et deux brins (3b) remontant le long de l'enveloppe (2) selon des enroulements hélicoïdaux de sens inverses, ces deux brins (3b) s'étendant de part et d'autre de la cheville (11), sensiblement au niveau des malléoles, se croisant sur l'avant de la jambe (14), sensiblement au niveau du tiers inférieur de celle-ci, se croisant ensuite sur l'arrière de la jambe (14) sensiblement à mi-hauteur ou au niveau du tiers supérieur de la jambe (14), passant ensuite de part et d'autre de l'articulation du genou (10), longeant la rotule, se croisant à nouveau sur l'avant de la cuisse (13), sensiblement au niveau du tiers inférieur de celle-ci, et se prolongeant ensuite au moins jusqu'à mi-cuisse (13).

## Description

La présente invention concerne un bas de contention destiné à être utilisé pour protéger ou soulager l'articulation du genou et/ou l'articulation de la cheville.

Ce bas peut notamment être utilisé lorsque l'une et/ou l'autre de ces articulations est(sont) fortement sollicitée(s), par exemple lors de la pratique de sports. Ce bas peut également être utilisé à des fins médicales, lorsque l'une et/ou l'autre de ces articulations présentent des faiblesses ou des pathologies, notamment ligamentaires. Tel est en particulier le cas à la suite d'une chirurgie ligamentaire du genou ou de la cheville.

Il existe des chevillères et des genouillères en tricot élastique, propres à être placées avec serrage autour de l'articulation concernée. Ces chevillères ou genouillères assurent une certaine protection et un certain soutien des articulations mais cette protection et ce soutien restent toutefois relativement limités et relativement peu efficaces. Ces chevillères ou genouillères ont également pour inconvénient d'induire une gêne relative dans le mouvement de l'articulation, ce qui peut être pénalisant pour la pratique de sports.

La présente invention vise à remédier à ces inconvénients essentiels, en fournissant un bas de contention propre à assurer une très bonne protection et un parfait soutien de l'articulation du genou et/ou de l'articulation de la cheville, sans induire une gêne notable dans le mouvement de ces articulations. Ce bas de contention a notamment pour objectif de créer une interdépendance de la cheville et du genou en ce qui concerne la réactivité aux contraintes.

Selon l'invention, ce bas de contention comprend :
- une enveloppe conformée pour recevoir étroitement le membre inférieur d'un utilisateur depuis le pied jusqu'à mi-cuisse au moins, et
- une bande de renforcement solidaire de l'enveloppe, comprenant une partie médiane qui passe, lorsque le bas est porté, sous le pied de l'utilisateur au niveau de la voûte plantaire, et deux brins remontant le long de l'enveloppe selon des enroulements hélicoïdaux de sens inverses, ces deux brins s'étendant, lorsque le bas est porté, de part et d'autre de la cheville, sensiblement au niveau des malléoles, se croisant sur l'avant de la jambe, sensiblement au niveau du tiers inférieur de celle-ci, se croisant ensuite sur l'arrière de la jambe sensiblement à mi-hauteur ou au niveau du tiers supérieur de la jambe, passant ensuite de part et d'autre de l'articulation du genou, longeant la rotule, se croisant à nouveau sur l'avant de la cuisse, sensiblement au niveau du tiers inférieur de celle-ci, et se prolongeant ensuite au moins jusqu'à mi-cuisse.

Le bas selon l'invention comprend ainsi une enveloppe propre à être engagée sur le membre inférieur de l'utilisateur pour positionner et maintenir ladite bande de renforcement par rapport à ce membre inférieur, et une bande de renforcement rapportée sur cette enveloppe, disposée de manière précise par rapport à des zones anatomiques déterminées de la jambe et de la cuisse.

Au niveau de la cheville, les portions de la bande de renforcement qui s'étendent le long des malléoles s'opposent efficacement au risque de torsion du pied en varus ou en valgus ; elles sont en effet disposées idéalement pour s'opposer à cette torsion, et sont parfaitement maintenues par rapport à la cheville du fait de la prise d'appui de l'enveloppe et de la bande de renforcement sur une large surface du membre inférieur.

Au niveau du genou, les portions des deux brins qui s'étendent de part et d'autre de l'articulation soutiennent latéralement cette articulation, de façon analogue auxdites portions de bande s'étendant au niveau des malléoles ; lorsque le membre est fléchi, ces mêmes portions des brins tendent à se déplacer vers l'arrière du membre étant donné leur croisement, d'une part, en arrière de la jambe et en dessous du genou et, d'autre part, en avant de la cuisse et au-dessus du genou. Ce déplacement a pour effet de mettre en tension la zone de l'enveloppe située en dessous du genou et sur l'avant de la jambe, ce qui opère une action de maintien de la jambe par rapport à la cuisse contre un risque de sub-luxation antérieure du tibia.

Par ailleurs, l'absence de bande de renforcement en avant et en arrière des articulations de la cheville et du genou permet au bas selon l'invention de ne pas gêner les mouvements du membre.

De préférence, la zone de l'enveloppe s'étendant, lorsque le bas est porté, en dessous de l'articulation du genou et sur l'avant de la jambe comprend des moyens de renforcement limitant l'extensibilité circonférentielle de l'enveloppe, c'est-à-dire dans le sens de l'écartement des portions des brins se trouvant au niveau de cette zone de l'enveloppe.

Ces moyens de renforcement augmentent ainsi le soutien de la jambe permettant de prévenir la sub-luxation du tibia.

Selon une forme de réalisation préférée de l'invention dans ce cas, les moyens de renforcement sont constitués par une bandelette extensible reliant l'une à l'autre les portions des brins se trouvant au niveau de ladite zone de l'enveloppe.

Avantageusement, l'enveloppe comprend des moyens pour son positionnement par rapport à la rotule du membre de l'utilisateur. Ces moyens peuvent notamment être constitués par un logement ou une ouverture aménagé(e) dans cette enveloppe, propre à recevoir la rotule.

Les portions de bande s'étendant au niveau du genou peuvent ainsi être précisément positionnées et maintenues par rapport à la rotule.

L'enveloppe peut être dimensionnée de manière à s'étendre au moins jusqu'en haut de la cuisse, et les deux brins de la bande de renforcement se croisent sensiblement au niveau du tiers supérieur de la cuisse, sur le côté arrière de celle-ci, ces brins se terminant respectivement au niveau de la zone interne et de la zone externe de la cuisse.

Le bas selon l'invention peut s'interrompre à mi-cuisse ou au sommet de la cuisse, comme indiqué ci-dessus, ou peut faire partie d'un collant comprenant une partie propre à envelopper le bassin de l'utilisateur.

Pour sa bonne compréhension, l'invention est à nouveau décrite ci-dessous en référence au dessin schématique annexé représentant, à titre d'exemple non limitatif, une forme de réalisation préférée du bas de contention qu'elle concerne.
La figure 1 en est une vue à plat, l'enveloppe 2 qu'il comprend étant représentée en pointillés et considérée comme transparente ;

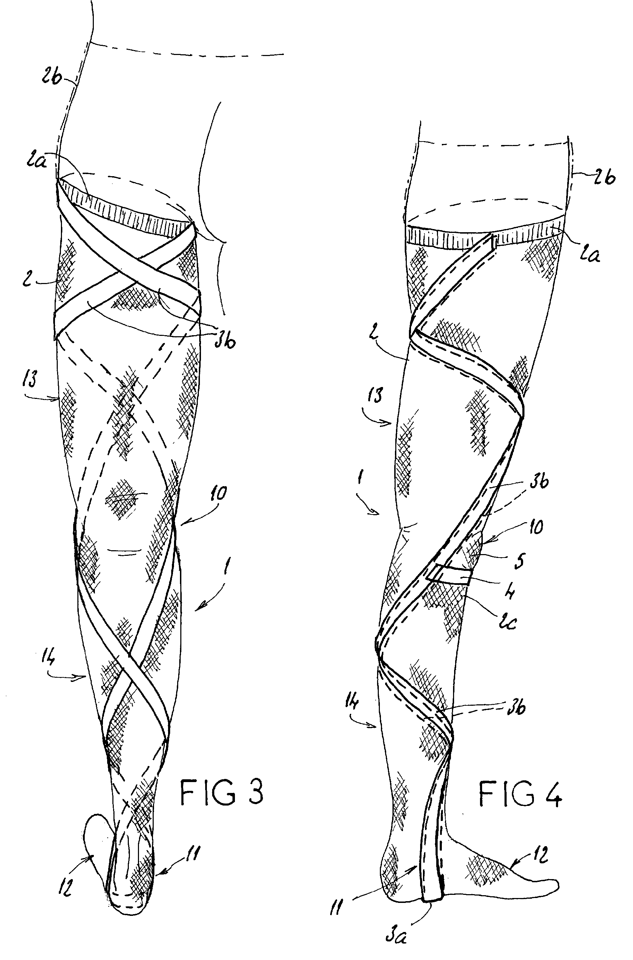

les figures 2 à 4 en sont des vues respectivement de face, de dos et de côté, alors qu'il est porté et que le membre inférieur est en extension, et
la figure 5 en est une vue de côté, alors qu'il est porté et que le membre inférieur est fléchi.

Les figures représentent un bas de contention 1 destiné à être utilisé pour protéger ou soulager l'articulation du genou 10 et/ou de la cheville 11 de l'utilisateur.

Le bas 1 comprend une enveloppe 2, une bande de renforcement 3 et une bandelette 4.

L'enveloppe 2 présente la forme d'une chausse conformée pour recevoir étroitement le membre inférieur d'un utilisateur depuis le pied 12 jusqu'en haut de la cuisse 13. A sa partie supérieure, l'enveloppe 2 peut comprendre une zone élastique 2a assurant son serrage autour de la cuisse 13.

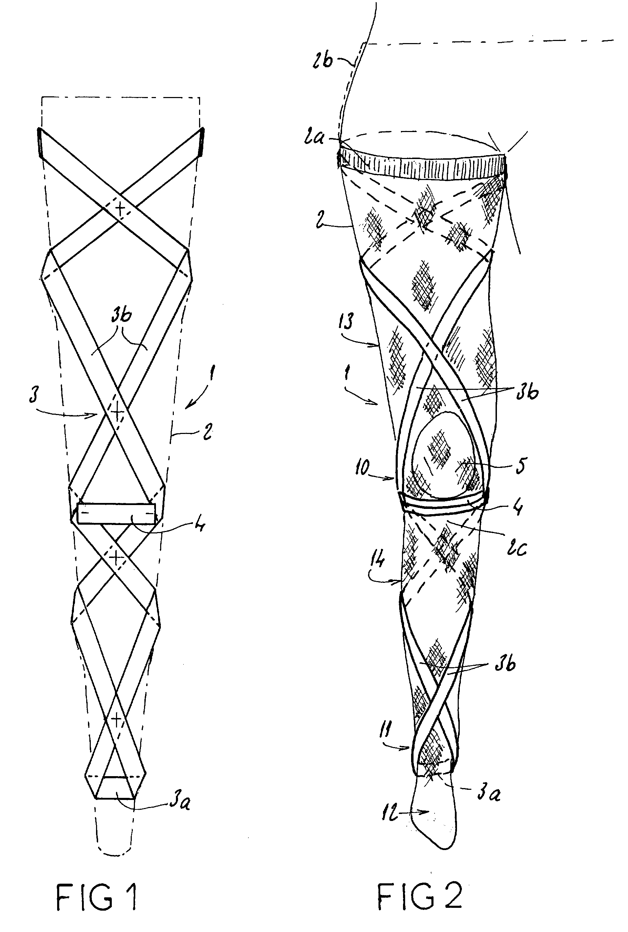

La figure 2 montre en pointillés que l'enveloppe 2 peut également faire partie d'un collant comprenant une partie 2b propre à envelopper le bassin de l'utilisateur.

L'enveloppe 2 comprend un logement 5 aménagé au niveau de sa zone destinée à venir en regard de la rotule de l'utilisateur, ce logement 5 étant dimensionné pour recevoir cette rotule en lui de manière ajustée.

La bande de renforcement 3 est solidaire de l'enveloppe 2. Il peut s'agir d'une bande textile faiblement élastique, cousue à l'enveloppe 2.

Ainsi que cela apparaît sur les figures, la bande 3 comprend une partie médiane 3a qui passe, lorsque le bas 1 est porté, sous le pied 12 de l'utilisateur au niveau de la voûte plantaire, et deux brins 3b remontant le long de l'enveloppe 2 selon des enroulements hélicoïdaux de sens inverses. Ces deux brins 3b sont fixés à l'enveloppe 2 de manière à s'étendre, lorsque le bas 1 est porté, de part et d'autre de la cheville 11, sensiblement au niveau des malléoles, à se croiser sur l'avant de la jambe 14, sensiblement au niveau du tiers inférieur de celle-ci, à se croiser ensuite sur l'arrière de la jambe 14 sensiblement au niveau du tiers supérieur de cette jambe, à passer ensuite de part et d'autre de l'articulation du genou 10, à longer la rotule, à se croiser à nouveau sur l'avant de la cuisse 13, sensiblement au niveau du tiers inférieur de celle-ci, puis sur l'arrière de la cuisse 13, sensiblement au niveau du tiers supérieur de celle-ci, ces brins 3b se prolongeant ensuite vers le haut pour se terminer respectivement au niveau de la zone interne supérieure et de la zone externe supérieure de la cuisse 13.

La bandelette 4 est en un matériau extensible. Elle peut avoir une même largeur que la bande 3 ou une largeur inférieure, comme représenté. Elle est cousue à l'enveloppe 2 au niveau de la zone 2c de celle-ci s'étendant, lorsque le bas 1 est porté, en dessous de l'articulation du genou 10 et sur l'avant de la jambe 14, et relie l'une à l'autre les portions des brins 3b se trouvant à sa hauteur, auxquelles elle est également cousue.

Comme cela résulte de ce qui précède, l'enveloppe 2 permet de positionner et de maintenir la bande 3 par rapport au membre inférieur sur laquelle elle est placée, de telle sorte que les brins 3b soient disposés de manière précise par rapport à des zones anatomiques déterminées de la jambe 14 et de la cuisse 13.

Le logement 5 forme un repère qui permet de bien positionner le bas 1 sur le membre, et en particulier de bien positionner et maintenir les portions de brins 3b ainsi que la bandelette 4 par rapport au genou 10 et à la rotule.

Au niveau du genou 10, les portions des deux brins 3b s'étendent, lorsque le membre est en extension, de part et d'autre de l'articulation et s'opposent ainsi efficacement au risque d'entorse du genou ; ces portions sont en effet disposées idéalement pour ce faire, et sont parfaitement maintenues par rapport au genou 10 du fait de la prise d'appui de l'enveloppe 2 et de la bande 3 sur une large surface du membre inférieur. Le calage angulaire et longitudinal de l'enveloppe 2 par rapport au membre, résultant de l'engagement de la rotule dans le logement 5, contribue à assurer le parfait positionnement de ces portions des brins 3b.

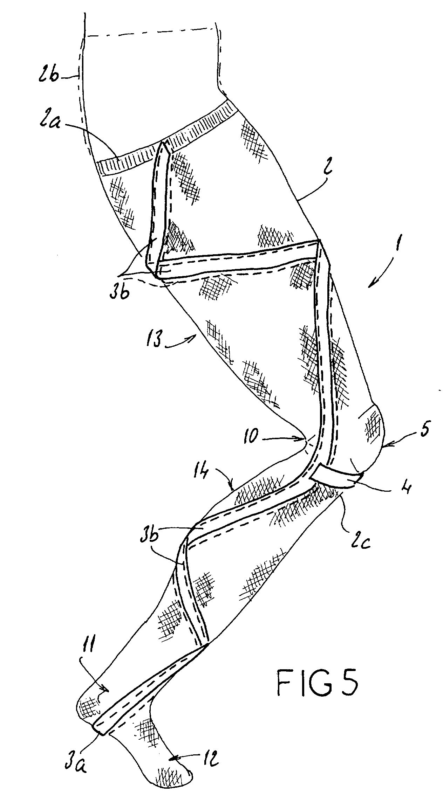

Lorsque le membre est fléchi, ces mêmes portions des brins 3b tendent à se déplacer vers l'arrière du membre (cf. figure 5) étant donné leur croisement, d'une part, en arrière de la jambe 14 et en dessous du genou 10 et, d'autre part, en avant de la cuisse 13 et au-dessus du genou 13. Ce déplacement vers l'arrière a pour effet d'étirer la bandelette 4, ce qui met cette dernière en tension et opère une action de maintien de la jambe 14 par rapport à la cuisse 13 contre un risque de sub-luxation antérieure du tibia.

Au niveau de la cheville 11, les portions de la bande 3 qui s'étendent le long des malléoles s'opposent efficacement au risque de torsion du pied en varus ou en valgus ; elles sont également disposées idéalement pour ce faire et sont également parfaitement maintenues par rapport à la cheville.

Par ailleurs, l'absence de bande 3 en avant et en arrière du genou 10 et de la cheville 11 permet au bas 1 de ne pas gêner les mouvements du membre, ce qui est avantageux notamment lors de la pratique de sports.

Ainsi qu'il apparaît de ce qui précède, l'invention apporte une amélioration déterminante à la technique antérieure, en fournissant un bas de contention 1 propre à assurer une très bonne protection et un parfait soutien de l'articulation du genou et/ou de l'articulation de la cheville, sans induire une gêne notable dans le mouvement de ces articulations. Ce bas peut dès lors être utilisé lorsque l'une et/ou l'autre de ces articulations sont fortement sollicitées, par exemple lors de la pratique de sports, ou à des fins médicales, lorsque l'une et/ou l'autre de ces articulations présentent des faiblesses ou des pathologies, notamment ligamentaires, ainsi que cela est par exemple le cas à la suite d'une chirurgie ligamentaire du genou ou de la cheville, ou en cas d'entorse du genou ou de la cheville.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées.

## Revendications

1. Bas de contention (1) destiné à être utilisé pour protéger ou soulager l'articulation du genou (10) et/ou l'articulation de la cheville (11), **caractérisé en ce qu'**il comprend :
- une enveloppe (2) conformée pour recevoir étroitement le membre inférieur d'un utilisateur depuis le pied (12) jusqu'à mi-cuisse (13) au moins, et
- une bande de renforcement (3) solidaire de l'enveloppe (2), comprenant une partie médiane (3a) qui passe, lorsque le bas (1) est porté, sous le pied (12) de l'utilisateur au niveau de la voûte plantaire, et deux brins (3b) remontant le long de l'enveloppe (2) selon des enroulements hélicoïdaux de sens inverses, ces deux brins (3b) s'étendant, lorsque le bas (1) est porté, de part et d'autre de la cheville (11), sensiblement au niveau des malléoles, se croisant sur l'avant de la jambe (14), sensiblement au niveau du tiers inférieur de celle-ci, se croisant ensuite sur l'arrière de la jambe (14) sensiblement à mi-hauteur ou au niveau du tiers supérieur de la jambe (14), passant ensuite de part et d'autre de l'articulation du genou (10), longeant la rotule, se croisant à nouveau sur l'avant de la cuisse (13), sensiblement au niveau du tiers inférieur de celle-ci, et se prolongeant ensuite au moins jusqu'à mi-cuisse (13).

2. Bas de contention (1) selon la revendication 1, **caractérisé en ce que** la zone (2c) de l'enveloppe (2) s'étendant, lorsque le bas (1) est porté, en dessous de l'articulation du genou (10) et sur l'avant de la jambe (14) comprend des moyens de renforcement (4) limitant l'extensibilité circonférentielle de l'enveloppe (2), c'est-à-dire dans le sens de l'écartement des portions des brins (3b) se trouvant au niveau de cette zone (2c) de l'enveloppe (2).

3. Bas de contention (1) selon la revendication 2, **caractérisé en ce que** les moyens de renforcement sont constitués par une bandelette (4) reliant l'une à l'autre les portions des brins (3b) se trouvant au niveau de ladite zone (2c) de l'enveloppe (2).

4. Bas de contention (1) selon l'une des revendications 1 à 3, **caractérisé en ce que** l'enveloppe (2) comprend des moyens (5) pour son positionnement par rapport à la rotule du membre de l'utilisateur.

5. Bas de contention (1) selon la revendication 4, **caractérisé en ce que** lesdits moyens de positionnement sont constitués par un logement (5) ou une ouverture aménagé(e) dans l'enveloppe (2), propre à recevoir la rotule.

6. Bas de contention (1) selon l'une des revendications 1 à 5, **caractérisé en ce que** l'enveloppe (2) est dimensionnée de manière à s'étendre au moins jusqu'en haut de la cuisse (13), et **en ce que** les deux brins (3b) de la bande de renforcement (3) se croisent sensiblement au niveau du tiers supérieur de la cuisse (13), sur le côté arrière de celle-ci, ces brins (3b) se terminant respectivement au niveau de la zone interne et de la zone externe de la cuisse (13).

7. Bas de contention (1) selon l'une des revendications 1 à 6, **caractérisé en ce que** l'enveloppe (2) comprend à sa partie supérieure une zone élastique assurant son serrage autour de la cuisse (13).

8. Bas de contention (1) selon l'une des revendications 1 à 6, **caractérisé en ce qu'**il fait partie d'un collant comprenant une partie (2b) propre à envelopper le bassin de l'utilisateur.
